# EUROPEAN PATENT APPLICATION

(11) **EP 2 420 518 A1**
(43) Date of publication of application: **22.02.2012**
(21) Application number: 10764133.4
(22) Date of filing: 14.04.2010
(51) Int. Cl.: C08B 37/16, C12N 15/88, A61K 31/7056

(54) **POLYCATIONIC AMPHIPHILIC CYCLOOLIGOSACCHARIDES AND THE USE THEREOF AS MOLECULAR TRANSPORTERS**

(30) Priority: 14.04.2009 ES 200900979
(71) Applicant: Consejo Superior de Investigaciones Cientificas (CSIC), 28006 Madrid (ES); Universidad De Sevilla, 41004 Sevilla (ES); Universidad De Granada (33,3%), 18071 Granada (ES)
(72) Inventor: ORTIZ MELLET, Carmen, E-41004 Sevilla (ES); MÉNDEZ ARDOY, Alejandro, E-41004 Sevilla (ES); GÓMEZ GARCÍA, Marta, E-41004 Sevilla (ES); SEVILLANO TRAPERO, Natalia, E-18071 Granada (ES); GIRÓN GONZÁLEZ, Mª Dolores, E-18071 Granada (ES); SALTO GONZÁLEZ, Rafael, E-18071 Granada (ES); SANTOYO GONZÁLEZ, Francisco, E-18071 Granada (ES); GARCÍA FERNÁNDEZ, José Manuel, E-41092 Sevilla (ES)
(74) Representative: Pons Ariño, Angel
(86) International application number: PCT/ES2010/070228
(87) International publication number: WO 2010/119158

(57) **Abstract**

The present invention relates to a group of polycationic amphiphilic cyclooligosaccharides, which, owing to the molecular structure thereof, can be used to transport molecules within cells. Furthermore, the present invention also relates to the use of said compounds in the preparation of a drug, to the use of this drug for gene therapy and to a pharmaceutical composition that comprises one of said compounds.

## Description

The present invention pertains to the field of molecular biology and biomedicine. Specifically, it relates to a group of polycationic amphiphilic cyclooligosaccharides, to the use thereof as transporters of molecules to the interior of cells, to the use of said compounds in the preparation of a medicament, to the use of this medicament for gene therapy and to a pharmaceutical composition that comprises one of said compounds.

### PRIOR STATE OF THE ART

The use of macrocyclic compounds as molecular templates to obtain a precise alignment of functional elements represents an interesting strategy for the design of artificial receptors or ligands capable of emulating the supramolecular events that take place in living organisms or of interfering with them. In this regard, cyclomaltooligosaccharides (cyclodextrins, CDs) are privileged macrocyclic platforms for these purposes, since they combine biocompatibility, availability, a symmetric tubular structure with well-differentiated faces, and are susceptible to controlled chemical functionalisation (Vargas-Berenguel et al. 2007. Mini-Rev. Org. Chem. 4, 1-14; García Fernández et al. 2006. J. Incl. Phenom. Macrocycl. yhyuyujjuChem. 56, 149-159). Moreover, cyclodextrin derivatives have the capacity to include other molecules in their inner cavity, to form inclusion complexes. For example, chemically modified CDs have been incorporated into polycationic polymers that are capable of effectively complexing and releasing DNA plasmids (pDNA) with a high biocompatibility and efficacy (Davis and Brewster. 2004. Nat. Rev. Drug. Discov. 3, 1023-1035). The surface of the corresponding particles may be subsequently modified by the non-covalent binding of chemical entities that may interact with the CD cavity.

The aforementioned examples highlight the interest of polycationic CD derivatives for the complexation and transport of other molecules, especially if they have an anionic nature. However, their polymeric and polydisperse character entails a significant limitation for the development of applications, particularly in the field of biomedicine. Some examples of CD derivatives with a molecular nature have been prepared by the functionalisation of the primary hydroxyls of natural cyclooligosaccharides, but their efficacy in protecting a biomolecule such as DNA and leading it to a target cell is relatively low (Srinivasacchari et al. 2008. J. Am. Chem. Soc. 130, 4618-4627; Mourtzis et al. 2008. Chem. Eur. J. 14, 4188-4200). Although said efficacy may be increased by providing polycationic CDs with an amphiphilic character (Díaz-Moscoso et al. 2008. Chem. Commun. 2001-2003), the preparation of this type of compounds is very limited, due to the low efficacy that multiple coupling methods generally have in CDs. Consequently, there is a need to develop new structures of polycationic amphiphilic cyclodextrins that may be prepared with a high efficacy and which are capable of effectively complexing and transporting other molecules, particularly including polyanionic biomolecules such as nucleic acids (DNA or RNA).

### DESCRIPTION OF THE INVENTION

The authors of the present invention have found a family of polycationic amphiphilic macrocyclic derivatives with properties that make them ideal vehicles to introduce molecules into cells.

A first aspect of the present invention relates to a compound with formula (I) 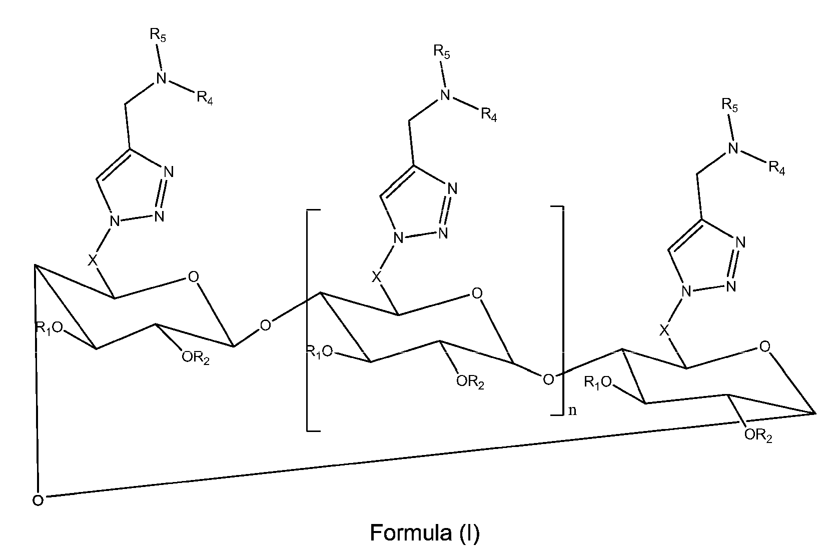 where:
n is 4, 5 or 6;
R¹ and R² are independently selected from hydrogen, C₁-C₂₀ alkyl or acyl; where at least one of R¹ and R² is other than H;
X is selected from -CH₂- or -CH₂YR³;
where Y is selected from O or S, R³ is -(CH₂)ₚ-NH-CO-(CH₂)_{q}-, where p and q may independently have a value ranging between 1 and 10.
R⁴ and R⁵ are independently selected from hydrogen and -(CH₂)ᵣ-[(NH)-(CH₂)ₛ]ₜ-NH-R₆; where r may have a value ranging between 1 and 10, s may have a value ranging between 2 and 10, and t may have a value ranging between 0 and 4,
R₆ is selected from H, a fluorochrome or a G group that comprises a ligand capable of being recognised by a cellular receptor,
or the organic or inorganic salts thereof.

In the present invention, the term "alkyl" refers to linear or branched hydrocarbon chain radicals, which consist of carbon and hydrogen atoms, which may or may not contain unsaturations, which have between one and twenty carbon atoms and are bound to the rest of the molecule by means of a single bond, for example, methyl, ethyl, n-propyl, i-propyl, n-butyl, t-butyl, n-pentyl, etc. The alkyl radicals may be optionally substituted with one or more substituents, such as an aryl, halogen, hydroxyl, alkoxyl, carboxyl, cyano, carbonyl, acyl, alkoxycarbonyl, amino, nitro, mercapto, alkylthio, etc.

In the present invention, the term "acyl" refers to a carboxylic acid derivative through the elimination of a hydroxyl group. Acyl groups have the general formula Ra-CO-, where Ra is an alkyl group with the above meanings, and preferably refers to alkyl groups (C₁-C₂₀), where alkyl is understood as defined above.

In the present invention, the term "cellular receptor" refers to a cell surface component that specifically recognises or interacts with a molecule or "ligand". Interaction of the ligand with the cellular receptor may favour, for example, selective distribution to a given target cell.

In a preferred embodiment, the present invention relates to a compound with formula (I), where R¹ and R² are C₂-C₁₄ alkyl or acyl.

In a preferred embodiment, the present invention relates to a compound with formula (I), where X es -CH₂-.

In another preferred embodiment, the present invention relates to a compound with formula (I), where X es -CH₂YR³, Y is sulfur, R³ is -(CH₂)ₚ-NH-CO-(CH₂)_{q}-, p is 2 and q is 1.

In a preferred embodiment, the present invention relates to a compound with formula (I), where R⁴ and R⁵ are -(CH₂)ᵣ-[(NH)-(CH₂)ₛ]ₜ-NH₂, where r has a value of 1, s has a value of 2 and t has a value ranging between 0 and 4.

In a preferred embodiment, the present invention relates to a compound with formula (I), where the salt thereof is selected from the group formed by acetate, trifluoroacetate, propionate, benzoate, methanesulfonate and trifluoromethanesulfonate.

In a preferred embodiment, the present invention relates to a compound with formula (I), where the salt thereof is selected from the group formed by chloride, bromide and iodide. More preferably, said salt is chloride.

In another preferred embodiment, the present invention relates to the compound with formula (I), where n is 5.

In a preferred embodiment, the present invention relates to a compound with formula (I), which is selected from the following group:
- Heptakis[6-(4-aminomethyl-1*H-*1,2,3-triazole-1-yl)-6-deoxy-2,3-di-*O-*hexanoyl]cyclomaltoheptaose
- Heptakis[6-(4-(2,2-diaminoethylaminomethyl)-1*H*-1,2,3-triazole-1-yl)-6-deoxy-2,3-di-*O*-hexanoyl]cyclomaltoheptaose
- Heptakis[6-(4-(2,2-diaminoethylaminomethyl)-1*H-*1,2,3-triazole-1-yl)-6-deoxy-2,3-di-*O*-myristoyl]cyclomaltoheptaose
- Heptakis[6-(2-(4-aminomethyl-1*H*-1,2,3-triazole-1-yl)acetamidoethylthio)-6-deoxy-2,3-di-*O*-hexanoyl]cyclomaltoheptaose
- Heptakis[6-(2-(4-(2,2-diaminoethylaminomethyl)-1*H*-1,2,3-triazole-1-yl)acetamidoethylthio)-6-deoxy-2,3-di-*O*-hexanoyl]cyclomaltoheptaose

In a preferred embodiment, the present invention relates to a compound with formula (I), where R₆ is a fluorochrome.

In the present invention, the term "fluorochrome" refers to a component of a molecule that makes the latter fluorescent. It is a functional group of the molecule that will absorb energy of a specific wavelength and re-emit it in another given higher wavelength (i.e. with a lower energy). The amount of energy emitted and the wavelength thereof are dependent on both the fluorochrome itself and its chemical environment. Some examples of fluorochromes that may be used in the present invention, without being limited thereto, are rhodamine, fluorescein or dansyl.

In another preferred embodiment, the present invention relates to a compound with formula (I), where R₆ is a G group that comprises a ligand selected from a mono- or an oligosaccharide, a monoclonal antibody or a folic acid derivative.

More preferably, the mono- or oligosaccharide is selected from mannose, galactose, glucose, N-acetylglucosamine, sialic acid, lactose or an oligosaccharide formed from any of them or the combinations thereof.

In the present invention, the term "monosaccharide" refers to the simplest glucosides, which are not hydrolysed, i.e. do not break down to give other compounds, and contain between three and six carbon atoms. Their empirical formula is (CH_{2O})*n*, where n ≥ 3. The monosaccharides' carbonated chain is not branched and all but one of the carbon atoms contain an alcohol group (-OH). The remaining carbon atom is bound to a carbonyl group (C=O). If this carbonyl group is at the end of the chain, it is an aldehyde group (-CHO) and the monosaccharide is called aldose. Examples of aldoses, without being limited thereto, are glyceraldehyde, erythrose, treose, ribose, arabinose, xylose, lyxose, allose, altrose, glucose, mannose, gulose, idose, galactose, talose. If the carbonyl carbon is at any other position, it is a ketone (-CO-) and the monosaccharide is called ketose. Examples of ketoses, without being limited thereto, are dihydroxyacetone, erythrulose, ribulose, xylulose, sicose, fructose, sorbose and tagatose.

In the present invention, the term "oligosaccharide" refers to a polymer formed from monosaccharides bound by O-glycosidic bonds, with a number of monomer units ranging between 3 and 10. Disaccharides are noteworthy amongst oligosaccharides; they are oligosaccharides formed by the union of two identical or different monosaccharides by means of an O-glycosidic bond (with the loss of a water molecule), mono- or dicarboxylic, which, moreover, may be α or β as a function of the hemiacetal or hemiketal -OH. The most common disaccharides are, without being limited thereto, glucose, fructose, lactose, maltose, isomaltose, trehallose and cellobiose.

In a more preferred embodiment, R₆ is a G group with the following formula:

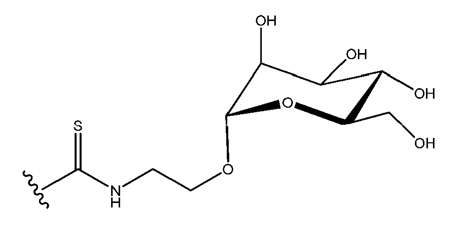

Another aspect of the invention relates to the use of the compound with formula (I) as a vehicle to transport, at least, one molecule to the interior of a cell.

As used in the present description, the term "transporter" or "carrier" refers to the capacity of the compound of the invention to transport a molecule to the interior of a cell. In Example 2 of the patent, a nucleic acid is used as a molecule to illustrate the potential of the compound of the invention to act as a cell-penetrating transporter. However, the molecule that may be introduced using the compound of the invention may be of a very diverse nature, for example, without being limited thereto, a nucleic acid, a peptide nucleic acid, a peptide, a protein, a glycoprotein, a carbohydrate, a lipid, a glycolipid, a fluorescent probe or a medicament. In Example 2, the DNA plasmid is administered to ovarian cells of wild-type Chinese hamsters (CHO-k1; no. ATCC CCL-61). However, the compound of the invention is useful to introduce a molecule into other types of eukaryotic cells and also prokaryotic cells.

A preferred embodiment of this aspect of the invention relates to the use of the compounds of the invention as transporters of nucleic acids to the interior of a cell.

The nucleic acid may be a deoxyribonucleic acid, such as, for example, without being limited thereto, double-chain DNA, single-chain DNA or circular DNA, or a ribonucleic acid, such as, for example, messenger RNA (mRNA) or signal interfering RNA (siRNA). The compound of the invention may be used to introduce more than one type of molecule, for example, two different DNA sequences.

In a more preferred embodiment, the nucleic acid is DNA. In an even more preferred embodiment, the DNA is double-chain DNA. In an even more preferred embodiment, the double-chain DNA is circular.

In another more preferred embodiment, the nucleic acid is RNA. In an even more preferred embodiment, the RNA is a signal interfering RNA (siRNA).

Nucleic acid transporters are also called vectors. As used in the present invention, the term "vector" refers to systems used in the process of transferring an exogenous nucleic acid to the interior of a cell, thereby allowing for the vehiculation of the nucleic acid to the interior of a cell.

Therefore, the compound of the invention may be used as a transformation or transfection agent. As used in the present description, the term "transformation" refers to the introduction of an exogenous nucleic acid into a prokaryotic cell. As used in the present description, the term "transformation agent" refers to a penetrating transporter of a nucleic acid to the interior of a prokaryotic cell. As used in the present description, the term "transfection" refers to the introduction of an exogenous nucleic acid into a eukaryotic cell. As used in the present description, the term "transfection agent" refers to a penetrating transporter of a nucleic acid to the interior of a eukaryotic cell.

A preferred embodiment of this aspect of the invention relates to the use of the compound with formula (I) as a transformation agent of nucleic acids to the interior of a prokaryotic cell.

Another preferred embodiment of this aspect of the invention relates to the use of the compound with formula (I) as a transfection agent of nucleic acids to the interior of a eukaryotic cell.

The compound of the invention may be used to transport, at least, one molecule with therapeutic capacity to the interior of a cell of an organism, preferably a mammal and, more preferably, a human being. Example 2 shows the capacity of the compound of the invention to introduce a nucleic acid into a eukaryotic cell. Therefore, the compound of the present invention is useful as a vector capable of transferring a prophylactic, diagnostic or therapeutic nucleic acid within the context of gene therapy.

Another aspect of the present invention relates to the use of the compound of the invention for the preparation of a medicament.

Another aspect of the present invention relates to the use of the medicament described above for gene therapy.

As used in the present description, the term "medicament for gene therapy" refers to a product obtained by means of a set of manufacturing processes aimed at transferring, either *in vivo* or *ex vivo,* a prophylactic, diagnostic or therapeutic nucleic acid to animal cells, preferably human, and the subsequent *in vivo* expression thereof. Gene transfer represents an expression system contained within a distribution system known as a vector, of viral or non-viral origin, which may likewise be included in an animal cell. Gene therapy medicaments include, without being limited thereto, the following: naked nucleic acids, non-viral vectors, viral vectors or genetically modified cells. They may be gene therapy medicaments based on autologous cells (from the patients themselves), as well as allogeneic (from another human being) or xenogeneic (from animals) cells.

Another aspect of the invention relates to a pharmaceutical composition that comprises the compound of the invention. In a preferred embodiment of this aspect of the invention, the pharmaceutical composition further comprises a pharmaceutically acceptable vehicle. In a more preferred embodiment of the present invention, the pharmaceutical composition further comprises, jointly with a pharmaceutically acceptable vehicle, another active principle.

As used herein, the terms "active principle", "active substance", "pharmaceutically active substance", "active ingredient" or "pharmaceutically active ingredient" refer to any component that potentially provides a pharmacological activity or another different effect in the diagnosis, cure, alleviation, treatment or prevention of a disease, or which affects the structure or the function of the body of human beings or other animals.

The pharmaceutical compositions of the invention may be formulated for administration in a variety of forms known in the state of the art. Such compositions and/or the formulations thereof may be administered to an animal and, more preferably, a mammal, including a human being, in a variety of forms, including, without being limited thereto, parenteral, intraperitoneal, intravenous, intradermal, epidural, intraspinal, intrastromal, intra-articular, intrasinovial, intrathecal, intralesional, intra-arterial, intracapsular, intracardiac, intramuscular, intranasal, intracraneal, subcutaneous, intraorbital or topical.

The dosage to obtain a therapeutically effective quantity is dependent on a variety of factors, such as, for example, age, weight, sex or tolerance of the animal. In the sense used in this description, the expression "therapeutically effective quantity" refers to the quantity of the pharmaceutically effective composition that produces the desired effect and, in general, will be determined, amongst other causes, by the characteristics of said pharmaceutical composition and the therapeutic effect to be achieved. The pharmaceutically acceptable "adjuvants" or "vehicles" that may be used in said compositions are the vehicles known in the state of the art.

Throughout the description and the claims, the word "comprises" and the variants thereof are not intended to exclude other technical characteristics, additives, components or steps. For persons skilled in the art, other objects, advantages and characteristics of the invention will arise partly from the description and partly from the practise of the invention. The following figures and examples are provided for illustrative purposes, and are not intended to limit the scope of the present invention.

### DESCRIPTION OF THE FIGURES

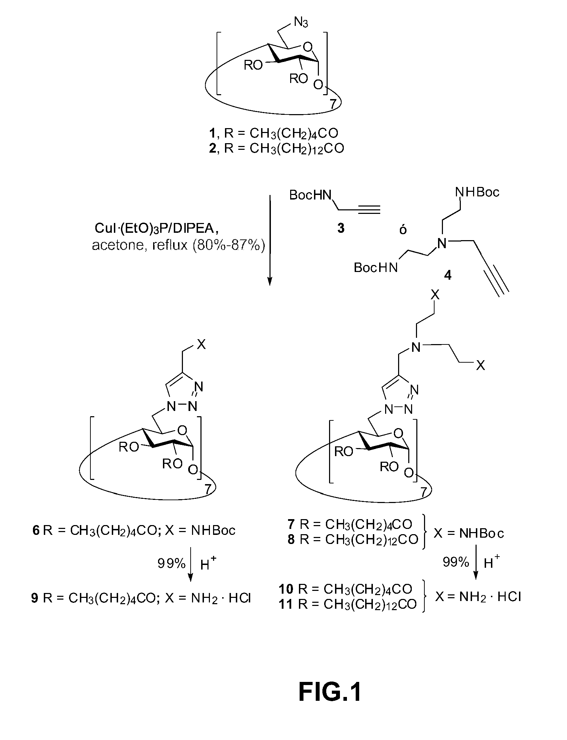

Figure 1.- Shows a schematic representation of the synthesis of rigid polycationic amphiphilic βCD derivatives **9-11.**

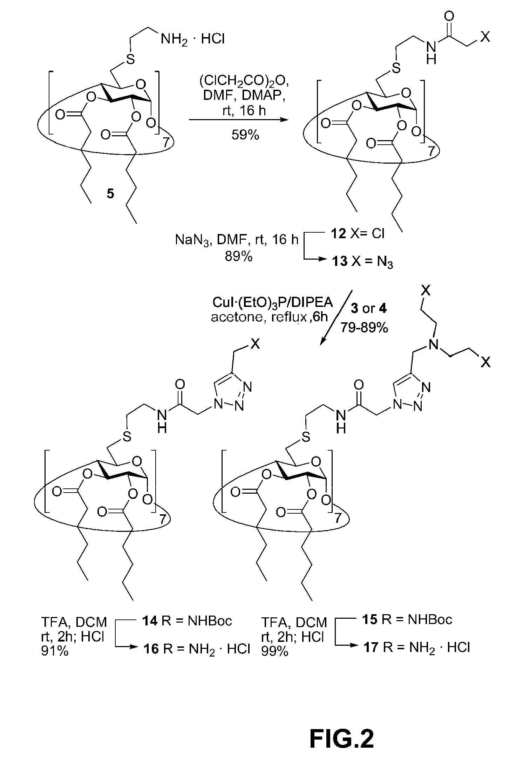

Figure 2.- Shows a schematic representation of the synthesis of flexible polycationic amphiphilic βCD derivatives **16** and **17.**
Figure 3.- Represents a gel electrophoresis shift assay that shows the binding of the amphiphilic (**9-11, 16** and **17)** and non-amphiphilic βCD derivatives (**18** and **19**) to the pDNA upon increasing the N/P ratios (a), and the quantification of the intensity of the bands (b).
Figure 4.- Shows the quantification of the relative intensity (value of untreated pDNA equal to 100) of the sum of the electrophoretic bands of relaxed and supertwisted pDNA corresponding to the pDNA samples complexed with polycationic amphiphilic cyclodextrins **9-11, 16** and **17** and treated with DNase I. The data represent the standard deviation of the mean (n = 4). The data for non-amphiphilic derivatives **18** and **19** are included for comparison purposes.
Figure 5.- Shows the efficacy of the *in vitro* gene transfection of the complexes of pDNA and **9-11, 16** and **17** in CHO-k1 cells, as compared to complexes derived from polycationic non-amphiphilic derivatives **(18** and **19),** polyplexes based on Lipofectamine^{™} 2000 (LP) and naked DNA (control). The data represent the standard deviation of the mean (n = 8).

### EXAMPLES

The following specific examples provided in this patent document are used to illustrate the nature of the present invention. These examples are included for illustrative purposes only and should not be interpreted as limitations to the invention claimed herein. Therefore, the examples described further below illustrate the invention without limiting the scope of application thereof.

### EXAMPLE 1: Chemical synthesis of the compounds.

**Materials.** The following derivatives, used in the preparation of the compounds of the invention, were prepared following methods described in the literature:
.- heptakis(6-azido-6-deoxy-2,3-di-*O*-hexanoyl)cyclomaltoheptaose (**1**) (Defaye 30 et al. WO2008009831).
.- heptakis(6-azido-6-deoxy-2,3-di-*O*-myristoyl)cyclomaltoheptaose (**2**) (Defaye et al. WO2008009831).
.- *N*-(*tert*-butoxycarbonyl)propargylamine (**3**) (Wu et al. 2007. J. Org. Chem. 72, 2897-2905).
.- bis[2-(tert-butoxycarbonylamino)ethyl]amine (Westerberg et al. 1989. J. Med. Chem. 32, 236-243), and
.- heptakis[6-(2-aminoethylthio)-6-deoxy-2,3-di-*O*-hexanoyl]-cyclomaltoheptaose heptahydrochloride (**5**) (Díaz-Moscoso et al. 2008. Chem. Commun. 2001-2003).

3-[Bis-2-(*tert*-butoxycarbonylamino)ethyl)amino]propyne (**4**) was prepared from bis[2-(*tert*-butoxycarbonylamino)ethyl]amine as shown below: propargyl bromide (0.90 ml, 8.6 mmol, 3.0 eq) was added to a solution of bis[2-(*tert*-butoxycarbonylamino)ethyl]amine (872 mg, 2.86 mmol) and Et₃N (1.2 ml, 8.6 mmol, 3.0 eq) in CH₃CN (15 ml), and the mixture was stirred at RT for 2 h. The solvent was eliminated under vacuum and the residue was dissolved in CH₂Cl₂ (100 ml), washed with water (3 × 30 ml), dried (MgSO₄) and concentrated. The resulting residue was purified by column chromatography (30:1 in CH₂Cl₂-MeOH). Yield: 567 mg (58%); *R*_{f} = 0.22 (50:1 CH₂Cl₂-MeOH); ¹H NMR (300 MHz, CDCl₃): δ 4.93 (bs, 2 H, NH), 3.42 (d, 2 H, ⁴*J*_{H,H} = 2.4 Hz, C*H*₂C=CH), 3.20 (q, 4 H, ³*J*_{H,H} - 5.7 Hz, C*H*₂NHBoc), 2.64 (t, 4 H, C*H*₂CH₂NHBoc), 2.20 (t, 1 H, C≡CH), 1.45 (s, 18 H, CMe₃); ¹³C NMR (75.5 MHz, CDCl₃): δ 156.0 (CO), 79.2 (Cq), 77.9 (*C*≡CH), 73.4 (C≡*C*H), 52.8 (*C*H₂CH₂NHBoc), 41.8 (HC=C*C*H₂), 38.0 (*C*H₂NHBoc), 28.4 (C*Me*₃). ESI-MS: *m*/*z* 364.2 [M + Na]⁺, 342.2 [M + H]⁺.

Plasmid pEGFP-N3 (GenBank registration no.: U57609) was obtained from Clontech Laboratories (Palo Alto, CA). This 4729-bp plasmid encodes a red-shifted wild-type GFP variant. The plasmid was purified from transfected bacteria using habitual processes (Sterzel et al. 1985. Anal. Biochem. 147, 462-467). The DNA concentration was measured by means of a fluorimetric process using the Hoechst 33258 stain.

### Preparation of precursors of the compounds of the invention with a rigid spacer (formula (I), X = CH₂), protected in the form of the corresponding tert-butyl carbamates

### Heptakis[6-(4-tert-butoxycarbonylami nomethyl-1H-1,2,3-triazole-1-yl)-6-deoxy-2,3-di-O-hexanoyl]cyclomaltoheptaose (6):

Cul·(EtO)₃P (71 mg, 200 µmol, 0.3 eq) and DIPEA (0.34 ml, 0.29 mmol, 1 eq) were added to a solution of 1 (765 mg, 0.28 mmol) and *N-tert-*butoxycarbonylpropargylamine (3, 405 mg, 2.59 mmol, 1.3 eq) in acetone (10 ml), and the reaction mixture was subjected to reflux for 3 h. The solvent was evaporated under reduced pressure and the resulting residue was purified by column chromatography (50:1 → 20:1 of CH₂Cl₂-MeOH). Yield: 871 mg (81%). *R*_{f} 0.47 (15:1 of CH₂Cl₂-MeOH); [α]_{D} = +24.7 (*c* 1.0, CHCl₃). ¹H NMR (500 MHz, CDCl₃, 313 K): δ 7.62 (s, 7 H, =CH), 5.45 (s, 7 H, H-1), 5.40 (s, 7 H, NHBoc), 5.38 (t, 7 H, *J*_{2,3} = *J*_{3,4} = 8.0 Hz, H-3), 4.86 (da, 7 H, *J*_{6a,6b} = 13.7 Hz, H-6a), 4.75 (dd, 7 H, *J*₁,₂ = 2.1 Hz, H-2), 4.57 (sa, 7 H, H-6b), 4.48 (sa, 7 H, H-5), 4.29 (dd, 7 H, *2J*_{H,H} = 15.2 Hz, *J*_{CH,NH} = 5.4 Hz, C*H*aNHBoc), 4.18 (dd, 7 H, *J*_{CH,NH} = 3.0 Hz, C*H*bNHBoc), 3.55 (t, 7 H, *J*₄,₅ = 8.0 Hz, H-4), 2.36-2.13 (m, 28 H, CH₂CO), 1.57 (m, 28 H, C*H*₂CH₂CO), 1.40 (s, 63 H, CMe₃), 1.30 (m, 56 H, C*H*₂CH₃, C*H*₂CH₂CH₃), 0.90 (t, 42 H, ³*J*_{H,H} = 7.0 Hz, CH₃). ¹³C NMR (125.7 MHz, CDCl₃, 313 K): δ 172.9, 171.6 (CO ester), 155.8 (CO carbamate), 145.2 (C-4 triazole), 124.6 (C-5 triazole), 96.4 (C-1), 79.5 (C-4, Cq), 70.1 (C-3), 69.8 (C-5), 69.6 (C-2), 50.2 (C-6), 36.1 (CH₂NHBoc), 33.8 (*C*H₂CO), 31.4, 31.2 (*C*H₂CH₂CH₃), 28.3 (C*M*e₃), 24.3 (*C*H₂CH₂CO), 22.3 (CH₂CH₃), 13.8 (CH₃). ESI-MS: *m*/*z* 1908.1 [M + 2 Na]²⁺.

### Heptakis[6-(4-(2,2-bis-tert-butoxycarbonylamino)ethylaminomethyl)-1H-1,2,3-triazole-1-yl)-6-deoxy-2,3-di-O-hexanoyl]cyclomaltoheptaose (7):

Cul·(EtO)₃P (15 mg, 41 µmol, 0.1 eq) was added to a solution of **1** (156 mg, 58 µmol) and 3-bis[2-(*tert*-butoxycarbonylamino)ethyl]aminopropyne (**4,** 182 mg, 0.53 mmol, 1.3 eq) in acetone (10 ml), and the reaction mixture was subjected to reflux for 3 h. The solvent was evaporated under vacuum and the residue was purified by column chromatography (30:1 → 9:1 of CH₂Cl₂-MeOH). Yield: 256 mg (87%); *R*_{f} = 0.61 (9:1 of CH₂Cl₂:MeOH); [α]_{D} = +25.9 (c 1.0, CH₂Cl₂); ¹H NMR (400 MHz, CDCl₃, 323 K): δ 7.73 (s, 7 H, =CH), 5.44 (d, 7 H, *J*_{1,2} = 3.2 Hz, H-1), 5.36 (t, 7 H, *J*_{2,3} = *J*_{3,4} = 8.7 Hz, H-3), 5.21 (sa, 14 H, NHBoc) 4.90 (da, 7 H, *J*_{6a,6b} = 13.6 Hz, H-6a), 4.75 (dd, 14 H, H-2, H-6b), 4.51 (m, 7 H, H-5), 3.78, 3.74 (2 d, 14 H, ²*J*_{H,H} = 15.6 Hz, CH₂N), 3.53 (t, 7 H, *J*_{4,5} = 8.5 Hz, H-4), 3.16 (q, 28 H, ³*J*_{H,H} = 5.6 Hz, C*H*₂NHBoc), 2.56 (t, 28 H, C*H*₂CH₂NHBoc), 2.45-2.13 (m, 28 H, CH₂CO), 1.60 (m, 28 H, C*H*₂CH₂CO), 1.44 (sa, 126 H, CMe₃), 1.30-1.20 (m, 56 H, C*H*₂CH₃, C*H*₂CH₂CH₃), 0.94, 0.93 (2 t, 42 H, ³*J*_{H,H} = 7.2 Hz, ³*J*_{H,H} = 6.8 Hz, CH₃); ¹³C NMR (100.6 MHz, CDCl₃, 323 K): δ 172.7, 171.5 (CO ester), 156.0 (CO carbamate), 143.6 (C-4 triazole), 125.4 (C-5 triazole), 96.5 (C-1), 78.8 (C-4, Cq), 70.0 (C-3), 69.7 (C-5), 69.3 (C-2), 53.0 (CH₂CH₂NHBoc), 50.0 (C-6), 48.0 (CH₂N), 38.5 (CH₂NHBoc), 33.8, 33.5 (CH₂CO), 31.1, 31.0 (CH₂CH₂CH₃), 28.3 (C*Me*₃), 24.1 (CH₂CH₂CO), 22.1 (CH₂CH₃), 13.6 (CH₃); ESI-MS: *m*/*z* 2558.2 [M + 2 Na]²⁺, 1713.6 [M + 3 Na]³⁺.

### Heptakis[6-(4-(2,2-bis(tert-butoxycarbonylamino)ethylaminomethyl)-1H-1,2,3-triazo)e-1-yl)-6-deoxy-2,3-di-O-myristoyl]cyclomaltoheptaose (8).

A solution of heptakis[6-azido-6-deoxy-2,3-di-O-myristoyl]cyclomaltoheptaose (**2,** 50 mg, 11 µmol), 3-bis[2-(*tert-*butoxycarbonylamino)ethyl]aminopropyne (**4,** 34 mg, 0.1 mmol, 1.3 eq), Cul(EtO)₃P (3 mg, 8 µmol, 0.1 eq) and *N*-ethyldiisopropylamine (13 µl, 80 µmol, 1 eq) in acetone (5 ml) was subjected to reflux for 6 h. The solvent was evaporated under reduced pressure and the residue was purified by column chromatography (30:1 → 9:1 of CH₂Cl₂:MeOH) to give an amorphous solid. Yield: 61.3 mg (80%). [α]_{D} = +26.2 (c 0.9, CH₂Cl₂); *R*_{f} = 0.51 (9:1 of CH₂Cl₂-MeOH); ¹H NMR (500 MHz, CDCl₃, 333 K): δ 7.28 (s, 7 H, =CH), 5.45 (s, 7 H, H-1), 5.38 (t, 7 H, *J*_{2,3} = *J*_{3,4} = 9.2 Hz, H-3), 5.19 (sa, 14 H, NHBoc), 4.90 (d, 7 H, *J*_{6a,6b} = 13.7 Hz, H-6a), 4.75 (dd, 14 H, *J*_{1,2} = 3.6 Hz, H-2, H-6b), 4.53 (m, 7 H, H-5), 3.78 (2d, 14 H, ²*J*_{Ha,Hb} = 15.7 Hz, CH₂N), 3.55 (t, 7 H, *J*_{4,5} = 8.1 Hz, H-4), 3.18 (qa, 28 H, ³*J*_{H,H} = 5.2 Hz, C*H*₂NHBoc), 2.58 (sa, 28 H, C*H*₂CH₂NHBoc), 2.44-2.15 (m, 28 H, CH₂CO), 1.60-1.54 (m, 28 H, C*H*₂CH₂CO), 1.46 (s, 126 H, CMe₃), 1.38-1.29 (m, 280 H, (CH₂)₁₀), 0.92 (t, 42 H, ³*J*_{H,H} = 6.5 Hz, CH₃); ¹³C NMR (125.7 MHz, CDCl₃, 313 K): δ 172.9, 171.7 (CO ester), 156.2 (CO carbamate), 143.6 (C-4 triazole), 125.7 (C-5 triazole), 96.6 (C-1), 79.0 (C-4, Cq), 70.1 (C-3), 69.7 (C-5, C-2), 53.1 (C*H*₂CH₂NHBoc), 50.1 (C-6), 48.0 (CH₂N), 38.4 (C*H*₂NHBoc), 34.0, 33.7 (CH₂CO), 31.9 (CH₃CH₂CH₂), 29.8-28.9 (CH₃CH₂CH₂(CH₂)₇), 28.5 (C*Me*₃), 24.6 (*C*H₂CH₂CO), 22.5 (CH₃CH₂), 14.0 (CH₃); ESI-MS: *m*/*z* 2228.6 [M + 2 Na + H]³⁺, 1681.2 [M +K + 2 Na + H]⁴⁺.
**General process for the preparation of polycationic amphiphilic macrocyclic derivatives with a rigid spacer, with formula (I), wherein X is CH₂ (compounds 9-11).** Treatment of the corresponding hepta- or tetradecacarbamate (**6-8**, 33 µmol) with TFA-CH₂Cl₂ (1:1, 2 ml) at room temperature for 2 h, followed by evaporation of the solvents and lyophilisation in a diluted HCl solution, gave pure **9-11.**

### Heptakis[6-(4-aminomethyl-1H-1,2,3-triazole-1-yl)-6-deoxy-2,3-di-O-hexanoyl]cyclomaltoheptaose heptahydrochloride (9):

Compound **9** corresponds to formula (I), wherein n = 5, the R¹ and R² groups are identical and represent hexanoyl (CH₃(CH₂)₄CO-), X is CH₂, and R⁴ and R⁵ are identical and represent hydrogen, in the form of the corresponding heptahydrochloride salt.
Yield: 106.4 mg (99%); [α]_{D} = +31.5 (c 1.0, MeOH); ¹H NMR (500 MHz, CD₃OD, 313 K): δ 7.98 (s, 7 H, =CH), 5.49 (t, 7 H, *J*_{2,3} = *J*_{3,4} = 9.3 Hz, H-3), 5.46 (s, 7 H, H-1), 4.88 (d, 7 H, *J*_{6a,6b} = 14.0 Hz, H-6a), 4.78 (dd, 7 H, *J*_{1,2} = 2.1 Hz, H-2), 4.65 (s, 7 H, H-6b), 4.53 (sa, 7 H, H-5), 3.91 (s, 14 H, C*H*₂NH₂), 3.67 (t, 7 H, *J*_{4,5} = 8.5 Hz, H-4), 2.47-2.21 (m, 28 H, CH₂CO), 1.64 (m, 28 H, C*H*₂CH₂CO), 1.36 (m, 56 H, C*H*₂CH₃, C*H*₂CH₂CH₃), 0.94 (t, 42 H, ³*J*_{H,H} = 7.0 Hz, CH₃); ¹³C NMR (125.7 MHz, CD₃OD, 313 K): δ 174.3, 173.4 (CO ester), 145.5 (C-4 triazole), 126.8 (C-5 triazole), 98.1 (C-1), 78.8 (C-4), 71.5 (C-5), 71.1 (C-3, C-2), 51.6 (C-6), 36.5 (CH₂NH₂), 35.0, 34.9 (CH₂CO), 32.5, 32.4 (CH₂CH₂CH₃), 25.5 (CH₂CH₂CO), 23.4 (CH₂CH₃), 14.2 (CH₃); (ESI)MS: m/z 1023.9 [M + 3 H]³⁺, 1535.3 [M + 2 H]²⁺_{.}

### Heptakis[6-(4-(2,2-diaminoethylaminomethyl)-1H-1,2,3-triazole-1-yl)-6-deoxy-2,3-di-O-hexanoyl]cyclomaltoheptaose tetradecahydrochloride (10):

Compound **10** corresponds to formula (I), wherein n = 5, the R¹ and R² groups are identical and represent hexanoyl (CH₃(CH₂)₄CO-), X is CH₂, and R³ and R⁴ are identical and represent 2-aminoethyl (NH₂(CH₂)₂-), in the form of the corresponding tetradecahydrochloride salt.
Yield: 136.4 mg (99%); [α]_{D} = +18.5 (c 1.0, MeOH); ¹H NMR (500 MHz, CD₃0D, 313 K): δ 8.11 (s, 7 H, =CH), 5.60 (t, 7 H, *J*_{2,3} = *J*_{3,4} = 9.9 Hz, H-3), 5.59 (s, 7 H, H-1), 5.15 (dd, 7 H, *J*_{6a,6b} = 15.2 Hz, *J*_{5,6a} = 2.5 Hz, H-6a), 4.58 (dd, 7 H, *J*_{5,6b} = 3.0 Hz, H-6b), 4.57 (m, 14 H, *J*_{1,2} = 3.5 Hz, H-2, H-5), 3.91, 3.89 (2 d, 14 H, ²*J*_{Ha,Hb} = 15.3 Hz, CH₂N), 3.54 (t, 7 H, *J*_{4,5} = 8.9 Hz, H-4), 3.15 (t, 28 H, ³*J*_{H,H} = 6.0 Hz, C*H*₂NH₂), 2.83 (t, 28 H, C*H*₂CH₂NH₂), 2.49-2.17 (m, 28 H, CH₂CO), 1.73-1.58 (m, 28 H, C*H*₂CH₂CO), 1.37 (m, 56 H, C*H*₂CH₃, C*H*₂CH₂CH₃), 0.94, 0.92 (2 t, 42 H, ³*J*_{H,H} = 7.1 Hz, ³*J*_{H,H} = 6.9 Hz, CH₃); ¹³C NMR (125.7 MHz, CD₃OD, 313 K): δ 175.7, 174.6 (CO ester), 144.6 (C-4 triazole), 129.7 (C-5 triazole), 99.2 (C-1), 79.4 (C-4), 73.0 (C-2), 72.9 (C-5), 71.7 (C-3), 53.0 (CH₂CH₂NH₂), 52.6 (C-6), 48.3 (CH₂N), 39.4 (CH₂NH₂), 36.3, 36.2 (CH₂CO), 33.9, 33.7 (CH₂CH₂CH₃), 26.8 (CH₂CH₂CO), 24.7 (CH₂CH₃), 15.6 (CH₃); ESI-MS: m/z 1837 [M + 2 H]²⁺, 1225.0 [M + 3 H]³⁺, 919.0 [M + 4 H]⁴⁺, 735.4 [M + 5 H]⁵⁺.

### Heptakis[6-(4-(2,2-diaminoethyl)aminomethyl)-1H-1,2,3-triazole-1-yl)-6-deoxy-2,3-di-O-myristoyl]cyclomaltoheptaose (11):

Compound **11** corresponds to formula (I), wherein n = 5, the R¹ and R² groups are identical and represent myristoyl (tetradecanoyl; CH₃(CH₂)₁₂CO-), X is CH₂, and R⁴ and R⁵ are identical and represent 2-aminoethyl (NH₂(CH₂)₂-), in the form of the corresponding tetradecahydrochloride salt.
Yield: 178.8 mg (99%); [α]_{D} = +30.7 (c 0.9, CH₂Cl₂); ¹H NMR (500 MHz, CD₃OD, 313 K): δ 8.12 (s, 7 H, =CH), 5.61 (t, 7 H, *J*_{2,3} = *J*_{3,4} = 9.3 Hz, H-3), 5.60 (s, 7 H, H-1), 5.13 (d, 7 H, *J*_{6a,6b} = 13.9 Hz, H-6a), 4.97 (d, 7 H, H-6b), 4.57 (m, 14 H, H-2, H-5), 3.90 (2d, 14 H, ²*J*_{H,H} = 15.3 Hz, CH₂N), 3.55 (t, 7 H, *J*_{4,5} = 9.2 Hz, H-4), 3.16 (t, 28 H, ³*J*_{H,H} = 5.5 Hz, C*H*₂NH₂), 2.82 (t, 28 H, C*H*₂CH₂NH₂), 2.50-2.15 (m, 28 H, CH₂CO), 1.80-1.55 (m, 28 H, C*H*₂CH₂C0), 1.50-1.25 (m, 280 H, CH₃ (C*H*₂)₁₀CH₂CH₂CO), 0.93 (t, 42 H, ³*J*_{H,H} = 6.6 Hz, CH₃); ¹³C NMR (125.7 MHz, CDCl₃, 313 K): δ 172.9, 171.8 (CO ester), 142.0 (C-4 triazole), 126.9 (C-5 triazole), 96.6 (C-1), 76.7 (C-4), 70.4 (C-5, C-2), 69.1 (C-3), 50.4 (*C*H₂CH₂NH₂), 49.9 (C-6), 45.7 (CH₂N), 36.7 (*C*H₂NH₂), 33.6 (CH₂CO), 31.7, 29.8-29.1, 22.3 (CH₃ (CH₂)₁₀CH₂CH₂CO), 24.6, 24.5 (CH₂CH₂CO), 13.0 (CH₃); ESI-MS: m/z 2622.0 [M + 2 H]²⁺, 1748.4 [M + 3H]³⁺, 1311.7 [M + 4 H]⁴⁺, 1049.7 [M + 5 H]⁵⁺.

### Preparation of precursors of the compounds of the invention with a flexible spacer, with formula (I), wherein X is -CH₂S(CH₂)₂- (compounds 12-15)

### Heptakis[6-(2-chloroacetamidoethylthio)-6-deoxy-2,3-di-O-hexanoyl]-cyclomaltoheptaose (12):

DMAP (270 mg, 2.24 mmol, 2 eq) was added in portions to a solution of heptahydrochloride 5 (500 mg, 0.16 mmol) in dry DMF (10 ml), under an Ar atmosphere, and the mixture was stirred for 15 min. Chloroacetic anhydride was added (570 mg, 3.36 mmol, 3 eq) and the reaction was further stirred overnight at RT. The solvent was evaporated under reduced pressure and the residue was dissolved in CH₂Cl₂, washed with slightly acidified water (3 x 20 ml), saturated aqueous NaHCO₃ (3 x 20 ml) and water (3 x 20 ml). The organic phase was dried (MgSO₄), filtered and concentrated. The residue was purified by column chromatography (30:1 of CH₂Cl₂-MeOH). Yield: 325 mg (59%); *R*_{f} = 0.53 (9:1 of CH₂Cl₂-MeOH); [α]_{D} = +80.3 (*c* 1.0, CH₂Cl₂); ¹H NMR (CDCl₃, 500 MHz, 313 K) δ 5.35 (t, 7 H, *J*_{2,3} = *J*_{3,4} = 9.5 Hz, H-3), 5.14 (d, 7 H, *J*_{1,2} = 4 Hz, H-1), 4.80 (dd, 7 H, H-2) 4.21 (m, 7 H, *J*_{4,5} = 9.0 Hz, *J*_{5,6a} = 2.5 Hz, *J*_{5,6b} = 5.0 Hz, H-5) 4.11 (2 d, 14 H, ²*J*_{H,H} = 15.0 Hz, CH₂Cl), 3.82 (t, 7H, H-4), 3.55 (m, 14 H, C*H*₂NH_{cyst}), 3.16 (dd, 7H, *J*_{6a,6b} = 14.0 Hz, H-6a), 3.10 (dd, 7H, H-6b), 2.85 (2dt, 14 H, ²*J*_{H,H} = 13_{.}5 Hz, ³*J*_{H,H} = 6.5 Hz, CH₂S_{cyst}), 2.43-2.17 (m, 28 H, CH₂CO), 1.70-1.57 (m, 28 H, C*H*₂CH₂CO), 1.36-1.29 (m, 56 H, C*H*₂CH₂CH₃, C*H*₂CH₃), 0.92 (2 t, 42 H, ³*J*_{H,H} = 7.0 Hz, ³*J*_{H,H} = 7.5 Hz, CH₃); ¹³C NMR (125.7 MHz, CDCl₃, 313 K) δ 173.3, 171.7 (CO ester), 166.5 (CO amide), 96.6 (C-1), 78.6 (C-4), 71.3 (C-5), 70.5 (C-2) 70.3 (C-3), 42.7 (CH₂Cl), 39.4 (CH₂NH_{cyst}), 34.1, 33.8 (*C*H₂CO, C-6), 32.9 (CH₂S_{cyst}), 31.4, 31.3 (*C*H₂CH₂CH₃), 24.3 (*C*H₂CH₂CO), 22.3 (*C*H₂CH₃), 13.8 (CH₃); ESI-MS: *m*/*z* 1175.3 [M + 3 Na]³⁺, 1752 [M + 2 Na]²⁺, 3477.7 [M + Na]⁺.

### Heptakis[6-(2-azidoacetamidoethylthio)-6-deoxy-2,3-di-O-hexanoyl]-cyclomaltoheptaose (13):

NaN₃ (43 mg, 0.66 mmol, 3 eq) was added to a solution of 12 (109 mg, 31 µmol) in dry DMF (10 ml), under an Ar atmosphere, and the mixture was stirred overnight. The solvent was eliminated under vacuum and the residue was dissolved in CH₂Cl₂ (50 ml), washed with H₂O (3 x 15 ml), dried (MgSO₄), filtered and concentrated. The resulting residue was purified by column chromatography (50:1 → 30:1 of CH₂Cl₂:MeOH). Yield: 93.2 mg (89%); *R*_{f} = 0.48 (9:1 of CH₂Cl₂:MeOH), [α]_{D} = +94.0 (*c* 1.0, CH₂Cl₂); ¹H NMR (CDCl₃, 300 MHz) δ 5.34 (t, 7 H, *J*_{2,3} = *J*_{3,4} = 9.6 Hz, H-3), 5.12 (d, 7 H, *J*_{1,2} = 3.6 Hz, H-1), 4.80 (dd, 7 H, H-2) 4.17 (m, 7 H, H-5) 4.02 (s, 14 H, CH₂N₃), 3.82 (t, 7 H, *J*_{4,5} = 8.7 Hz, H-4) 3.51 (m, 14 H, CH₂NH_{cyst}), 3.10 (m, 14 H, H-6a, H-6b), 2.82 (2 dt, 14 H, ²*J*_{H,H} = 13.8 Hz, ³*J*_{H,H} = 7.2 Hz, CH₂S_{cyst}), 2.44-2.12 (m, 28 H, CH₂CO) 1.62-1.55 (m, 28 H, C*H*₂CH₂CO), 1.34-1.30 (m, 56 H, C*H*₂CH₂CH₃, C*H*₂CH₃), 0.92 (2 t, 42 H, ³*J*_{H,H} = 11.1 Hz, ³*J*_{H,H} = 11.4 Hz, CH₃); ¹³C NMR (75.5 MHz, CDCl₃, 313 K) δ 173.7, 172.0 (CO ester), 167.7 (CO amide), 96.7 (C-1), 78.8 (C-4), 71.5 (C-5), 70.8 (C-2), 70.5 (C-3), 52.8 (CH₂N₃), 39.2 (CH₂NH_{cyst}), 34.3, 34.1 (*C*H₂CO), 33.8 (C-6), 33.1 (CH₂S_{cyst}), 31.7, 31.6 (*C*H₂CH₂CH₃), 24.7 (*C*H₂CH₂CO), 22.7 (*C*H₂CH₃), 14.2 (CH₃); ESI-MS: *m*/*z* 1774.6 [M + 2 Na]²⁺.

### Heptakis[6(2-(4-(N-tert-butoxycarbonyl)aminomethyl)-1H-1,2,3-triazole-1-yl)acetamidoethylthio)-6-deoxy-2,3-di-O-hexanoyl]cyclomaltoheptaose (14):

(EtO)₃P·Cul (3 mg, 9 µmol, 0.1 eq) and DIPEA (15 µl, 90 µmol, 1 eq) were added to a solution of **13** (45 mg, 13 µmol) and **3** (40 mg, 0.12 mmol, 1.3 eq) in acetone (5 ml), and the mixture was subjected to reflux for 4 h. The solvent was evaporated under reduced pressure and the residue was purified by column chromatography (20:1 → 9:1 of CH₂Cl₂:MeOH). Yield: 53 mg (89%); *R*_{f} = 0.4 (9:1 of CH₂Cl₂:MeOH); [α]_{D} = +67.6 (c 1.0, H₂Cl₂); ¹H NMR (CD₃OD, 500 MHz, 323 K) δ 7.90 (s, 7 H, =CH), 5.32 (t, 7 H, *J*_{2,3} = *J*_{3,4} = 8.5 Hz, H-3), 5.15 (d,7 H, *J*_{1,2} = 3.6 Hz, H-1), 5.13 (s, 14 H, C*H*₂CONH), 4.87 (dd, 7 H, H-2), 4.34 (s, 14 H, C*H*₂NHBoc), 4.28 (m, 7 H, H-5), 3.84 (t, 7 H, *J*_{4,5} = 8.0 Hz, H-4), 3.54 (m, 14 H, C*H*₂NH_{cyst}), 3.40 (m, 7 H, H-6a), 3.08 (dd, 7 H, *J*_{6a,6b} = 13.9 Hz, *J*_{5,6b =} 7.0 Hz, H-6b), 2.88 (m, 14 H, CH₂S_{cyst}), 2.50-2.22 (m, 28 H, CH₂CO), 1.70-1.57 (m, 28 H, C*H*₂CH₂CO), 1.42 (s, 63 H, CMe₃), 1.40-1.30 (m, 56 H, C*H*₂CH₂CH₃, C*H*₂CH₃), 0.96, 0.95 (2 t, 42 H, ³*J*_{H,H} = 7.0 Hz, CH₃); ¹³C NMR (125.7 MHz, CD₃OD): δ 174.5, 173.3 (CO ester), 167.9 (CO amide), 158.1 (CO carbamate), 147.4 (C-4 triazole), 125.7 (C-5 triazole), 98.3 (C-1), 80.6 (C-4, Cq), 72.9 (C-5), 72.1 (C-3), 71.6 (C-2), 53.4 (CH₂CONH), 40.7 (CH₂NH_{cyst}), 37.0 (CH₂NHBoc), 35.1 (CH₂CO), 32.6, 32.5 (CH₂CH₂CH₃, CH₂S_{cyst}, C-6), 28.9 (C*M*e₃), 25.5 (CH₂CH₂CO), 23.4 (*C*H₂CH₃), 14.4 (CH₃); ESI-MS: (*m*/*z*) 4606.9 [M + Na]⁺, 2317.9 [M + 2 Na]²⁺, 1552.2 [M + 3 Na]³⁺.

### Heptakis[6-(2-(4-(2,2-bis(tert-butoxycarbony)amino)ethy)aminomethyl)-1H-1,2,3-triazole-1-yl)acetamidoethylthio-6-deoxy-2,3-di-O-hexanoyl]cyclomaltoheptaose (15):

Cul·(EtO)₃P (4 mg, 10 µmol, 0.1 eq) and DIPEA (17 µl, 0.1 mmol, 1 eq) were added to a solution of **13** (48 mg, 14 µmol) and **4** (44 mg, 0.13 mmol, 1.3 eq) in acetone (5 ml), and the reaction mixture was subjected to reflux for 3 h. The solvent was eliminated under vacuum and the residue was purified by column chromatography (30:1 → 9:1 of CH₂Cl₂-MeOH) to give 15. Yield: 65 mg (79%); *R*_{f} = 0.36 (9:1 of CH₂Cl₂:MeOH); [α]_{D} = +51.5 (c 1.0, CH₂C1₂); ¹H NMR (500 MHz, CDCl₃, 313 K): δ 7.81 (s, 7 H, =CH), 5.34 (s, 14 H, NHBoc) 5.26 (t, 7 H, *J*_{2,3} = *J*_{3,4} = 7.9 Hz, H-3), 5.10 (m, 21 H, C*H*₂CONH, H-1), 4.76 (da, 7 H, H-2), 4.14 (sa, 7 H, H-5), 3.81 (sa, 14 H, C*H*₂N), 3.72 (ta, 7H, *J*_{4,5} = 6.95 Hz, H-4), 3.44 (m, 14 H, C*H*₂NH_{cyst}), 3.18 (sa, 14 H, C*H*₂NHBoc), 2.98 (sa, 7 H, H-6a), 2.80 (sa, 14 H, CH₂S_{cyst}), 2.73 (sa, 7 H, H-6b), 2.58 (s, 14 H, C*H*₂CH₂NHBoc), 2.42-2.10 (m, 28 H, CH₂CO), 1.65-1.50 (m, 28 H, C*H*₂CH₂CO), 1.42 (s, 126 H, CMe₃), 1.38-1.20 (m, 56 H, C*H*₂CH₂CH₃, C*H*₂CH₃), 0.95- 0.84 (2 t, 42 H, ³*J*_{H,H} = 7.3 Hz, ³*J*_{H,H} = 7.0 Hz, CH₃); ¹³C NMR (125.7 MHz, CDCl₃, 313 K): δ 173.4, 171.6 (CO ester), 166.0 (CO amide), 156.3 (CO carbamate) 144.1 (C-4 triazole), 125.1 (C-5 triazole), 96.6 (C-1), 79.1 (C-4, Cq), 71.4 (C-5), 70.4 (C-3, C-2), 53.3 (*C*H₂CONH), 52.4 (*C*H₂CH₂NHBoc), 48.3 (CH₂N), 39.4 (CH₂NH_{cyst}), 38.4 (CH₂NHBoc) 34.0, 33.8 (*C*H₂CO), 32.9 (C-6, CH₂S_{cyst}), 31.3, 31.2 (*C*H₂CH₂CH₃), 28.5 (C*Me*₃), 24.3 (*C*H₂CH₂CO), 22.3 (*C*H₂CH₃), 13.8 (CH₃); ESI-MS: *m*/*z* 2969.7 [M + 2 Na]²⁺, 2959.0 [M+ H + Na]²⁺.
**General process for the preparation of polycationic amphiphilic macrocyclic derivatives with a flexible spacer, with formula (I), wherein X is -CH₂S(CH₂)₂₋ (compounds 16 and 17). Treatment** of the corresponding hepta- or tetradecacarbamate (**14** or **15,** 19 µmol) with TFA-CH₂Cl₂ (1:1, 2 ml) at RT for 2 h, followed by evaporation of the solvents and lyophilisation in a diluted HCl solution, gave pure **16 and 17.**

### Heptakis[6(2-(4-aminomethyl-1H-1,2,3-triazole-1-yl)acetamidoethylthio)-6-deoxy-2,3-di-O-hexanoyl]cyclomaltoheptaose (16):

Compound **16** corresponds to formula (I), wherein n = 5, the R¹ and R² groups are identical and represent hexanoyl (CH₃(CH₂)₄CO-), X is - CH₂S(CH₂)₂-, and R⁴ and R⁵ are identical and represent hydrogen, in the form of the corresponding heptahydrochloride salt.
Yield: 72 mg (91%); [α]_{D} = +22.1 (*c* 0.8, MeOH); ¹H NMR (500 MHz, CD₃OD, 323 K): δ 8.21 (s, 7 H, =CH), 5.34 (t, 7 H, *J*_{2,3} = *J*_{3,4} = 7.9 Hz, H-3), 5.26 (s, 14 H, C*H*₂CONH), 5.16 (d, 7 H, *J*_{1,2} = 3.2 Hz, H-1), 4.85 (dd, 7 H, H-2), 4.33 (s, 14 H, C*H*₂NH₂), 4.29 (sa, 7 H, H-5), 3.87 (t, 7 H, *J*_{4,5} = 7.9 Hz, H-4), 3.54 (m, 14 H, C*H*₂NH_{cyst}), 3.42 (da, 7 H, *J*_{6a,6b} = 11.4 Hz, H-6a), 3.12 (dd, 7 H, *J*_{5,6b} = 4.3 Hz, H-6b), 2.91 (m, 14 H, CH₂S_{cyst}), 2.50-2.20 (m, 28 H, CH₂CO), 1.65 (m, 28 H, C*H*₂CH₂CO), 1.45-1.30 (m, 56 H, C*H*₂CH₂CH₃, C*H*₂CH₃), 1.00-0.92 (2 t, 42 H, ³*J*_{H,H} = 7.3 Hz, ³*J*_{H,H} = 6.9 Hz, CH₃); ¹³C NMR (100.3 MHz, CD₃OD, 313 K): δ 173.3, 171.9 (CO ester), 166.3 (CO amide), 139.7 (C-4 triazole), 126.0 (C-5 triazole), 96.8 (C-1), 79.2 (C-4), 71.2 (C-5), 70.4 (C-3), 70.1 (C-2), 51.8 (*C*H₂CONH), 39.1 (CH₂NH_{cyst}), 34.9 (CH₂NH₂), 33.6 (CH₂CO, CH₂S_{cyst}, C-6), 31.5, 31.1 (*C*H₂CH₂CH₃), 24.1 (*C*H₂CH₂CO), 22.0 (*C*H₂CH₃), 13.0, 12.8 (CH₃). ESI-MS: *m*/*z* 1976.6 [M + 2 Na]²⁺, 1317.8 [M + 3 Na]³⁺, 988.1 [M + 2 H + 2 Na]⁴⁺.

### Heptakis[6(2-(4-(2,2-diaminoethylaminomethyl)-1H-1,2,3-triazole-1-yl)acetamidoethylthio)-6-deoxy-2,3-di-O-hexanoyl]cyclomaltoheptaose (17):

Compound **17** corresponds to formula (I), wherein n = 5, the R¹ and R² groups are identical and represent hexanoyl (CH₃(CH₂)₄CO-), X is -CH₂S(CH₂)₂-, and R⁴ and R⁵ are identical and represent 2-aminoethyl (NH₂(CH₂)₂-), in the form of the corresponding tetradecahydrochloride salt.
Yield: 81.0 mg (91%). [α]_{D} = +39.6 (c 1.0 in MeOH); ¹H NMR (500 MHz, CD₃OD, 313 K): δ 8.05 (s, 7 H, =CH), 5.36 (t, 7 H, *J*_{2,3} = *J*_{3,4} = 8.7 Hz, H-3) 5.21 (s, 14 H, C*H*₂CONH), 5.17 (d, 7 H, *J*_{1,2} = 3.5 Hz, H-1), 4.83 (dd, 7 H, H-2), 4.21 (sa, 7 H, H-5), 3.90 (sa, 21 H, CH₂N, H-4), 3.52 (m, 14 H, C*H*₂NH_{cyst}), 3.15 (t, 14 H, C*H*₂NH₂, ³*J*_{H,H} = 5.5 Hz), 3.11 (m, 7 H, H-6a), 2.88 (m, 21 H, CH₂S_{cyst}, H-6b), 2.84 (t, 7 H, C*H*₂CH₂NH₂), 2.50-2.22 (m, 28 H, CH₂CO), 1.73-1.58 (m, 28 H, C*H*₂CH₂CO), 1.43-1.30 (m, 56 H, C*H*₂CH₃), 0.99-0.91 (2 t, 42 H, ³*J*_{H,H} = 6.9 Hz, ³*J*_{H,H} = 5.8 Hz, CH₃); ¹³C NMR (125.7 MHz, CD₃OD, 313 K): δ 176.0, 174.7 (CO ester), 169.2 (CO amide), 145.4 (C-4 triazole), 128.5 (C-5 triazole), 99.5 (C-1), 81.8 (C-4), 74.5 (C-5), 73.0 (C-3, C-2), 54.4 (*C*H₂CONH), 53.4 (*C*H₂CH₂NH₂), 49.1 (*C*H₂N), 42.0 (CH₂NH_{cyst}), 39.6 (*C*H₂NH₂) 36.4, 36.3 (*C*H₂CO), 33.8, 33.7 (*C*H₂CH₂CH₃), 32.0 (C-6, CH₂S_{cyst}) 26.8 (*C*H₂CH₂CO), 24.7 (CH₂CH₃), 15.7, 15.6 (CH₃).
**Incorporation of mannose units as ligands biorecognisable by the mannose/fucose receptor present in the cell wall of macrophages.**

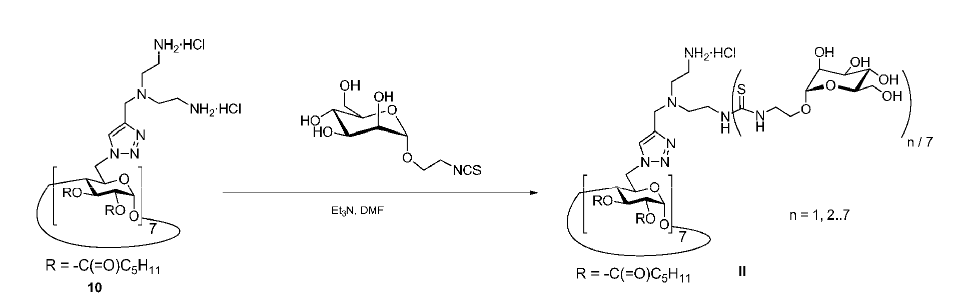

A solution of 2-ethyl-isothiocyanate α-D5-mannopyranoside (2.2 mg, 6.7 mg, 13.3 mg or 22.3 mg, which correspond to 0.05, 0.15, 0.30 or 0.50 equivalents relative to the NH₂ groups on **10)** in DMF (5 ml) was added to a solution of compound **10** (50 mg, 12 µmol) and Et₃N (47 µl, 0.34 mmol, 2 eq) in dry DMF (5 ml), and the mixture was stirred at room temperature overnight. Once this time had elapsed, the solvent was eliminated under vacuum and the resulting residue was purified by gel-exclusion chromatography (Sepadex G-25) using water as the eluent. The purified product was diluted with 0.1 N HCI and lyophilised, to obtain the corresponding adducts that incorporate mannose, as a white foam. ¹H NMR (500 MHz, D₂O, 323 K) δ 8.60-8.40 (m, =CH), 5.80-5.60 (m, H-1, H-3), 5.30-5.00 (m, H-6a, H-6b), 5.00-4.50 (H-2, H-5, H-1'), 4.20-3.60 (CH₂-triazole, H-4, H-2', CHaO, H-6a', H-3', CHbO, CH₂NHCO, H-6b', H-4m, H-5'), 3.50-2.90 (C*H*₂NH₂, C*H*₂CH₂NH₂), 2.70-2.30 (COCH₂), 1.79 (COCH₂C*H*₂), 1.50 (C*H*₂C*H*₂CH₃), 1.07 (CH₃).

ESI-MS data (the number of mannose units, "m", incorporated as a function of the percentage of reagent added is indicated)

For 5% of reagent, ESI-MS: m/z 735.6 [M + 5 H]⁵⁺, 918.8 [M + 4 H]⁴⁺, 944.6 [M + 2 K + Na + H]⁴⁺, 985.3 [M(1m) + 5 H]⁵⁺, 1051.8 [M(2m) + 4 H]⁴⁺, 1225 [M + 3 H]³⁺, 1313.4 [M(1m) + 3 H]³⁺.

For 15% of reagent, ESI-MS: m/z 735.4 [M+ 5 H]⁵⁺, 1052 [M(2m) + 4 H]⁴⁺, 1118.1 [M(3m) + 4 H]⁴⁺, 1401.7 [M(2m) + 3 H]³⁺, 1490.1 [M(3m)+ 3 H]³⁺.

For 30% of reagent, ESI-MS: m/z [M(3m) + 5 H]⁵⁺, 946.9 [M(4m) + 5 H]⁵⁺, 1051 [M(2m) + 4 H]⁴⁺, 1116.5 [M(3m) + 4 H]⁴⁺, 1183.1 [M(4m)+ 4 H]⁴⁺, 1249.6 [M(5m) + 4 H]⁴⁺, 1400.2 [M(2m) +3 H]³⁺, 1577 [M(4m) + 3 H]³⁺, 1664.8 [M(5m) + 3 H]³⁺.

For 50% of reagent, ESI-MS: m/z [M(6m) + 5 H]⁵⁺, 1106.9 [M(7m) + 5 H]⁵⁺, [M(6m) + 4 H]⁴⁺, 1382.9 [M(7m) + 4H]⁴⁺, 1449.4[M(8m) + 4H]⁴⁺, 1515.9 [M(9m) + 4H]⁴⁺_{.}

### EXAMPLE 2: Preparation of the complexes between polycationic amphiphilic macrocycles and plasmid DNA.

**Preparation of complexes from polycationic amphiphilic βCD derivatives and plasmid pEGFP-N3**. Plasmid pEGFP-N3, used for the preparation of the DNA complexes and the transfection assay, is a 4729-bp (base pairs) plasmid. The quantities of polycationic amphiphilic βCD derivatives used were calculated on the basis of the desired DNA concentration, 0.02 mg/ml (50 µM phosphate), the N/P ratio, the molecular weight and the number of positive charges in the selected CD derivative. The experiments were performed for N/P 5, 10, 30 and 50. The DNA was diluted in milli-Q water to a final phosphate concentration of 50 µM; subsequently, the desired quantity of CD derivative was added from a 20-mg/ml solution (1:2 of Me₂SO-water). The preparation was stirred with a vortex for a few seconds and incubated for 30 minutes.

### EXAMPLE 3: Analysis of the properties of the new polycationic CDs.

The capacity of the new polycationic amphiphilic CDs to complexate, protect and transfect the pDNA was analysed by means of gel electrophoresis shift assays, protection assay against the action of DNase and analysis of the transfection efficiency of plasmid pEGFP-N3 in eukaryotic cells, described in detail below.

**Gel electrophoresis lag-time assay. The binding capacity of the** polycationic amphiphilic DNA-βCD complex was analysed by gel electrophoresis. The stock solutions of βCD derivatives were prepared in 1:2 Me₂SO-water, and the DNA was dissolved in 150 mM NaCI. The DNA of pEGFP-N3 (10 µl at 0.1 µg/µl) was mixed with an equal volume of βCD derivative using N/P ratios ranging between 0 and 10, and incubated for 30 min at room temperature prior to the addition of loading buffer (2 µl). One aliquot (5 µl) of each sample was subjected to agarose gel electrophoresis (0.8% w/v) in TAE buffer (40 mM Tris-acetate, 1 mM EDTA). The electrophoresis was performed at 7 V/cm and the gels were stained with ethidium bromide following the electrophoresis. The quantification of the intensity of the bands was performed with the NIH Image software (Rasband et al. 1995. Microbeam Analysis Soc. J. 4, 137-149). A value of 100 was assigned to the intensity of the band for naked DNA.

**DNase protection assays.** The DNA of pEGFP-N3 (10 µl at 0.1 µg/µl) was mixed with an equal volume of stock solution of βCD derivative using N/P = 5, and incubated for 30 min at room temperature. 15 µl of a DNase I solution (0.1 mg/ml in 50 mM Tris-HCl, pH 8, 2000 U/mg) were added to the mixture and it was incubated for 45 minutes at 37ºC. Following the digestion, 20 µl of a 10% SDS solution were added and the samples were incubated for 2 h at 85ºC. Finally, one aliquot (20 µl) of each sample was subjected to agarose gel electrophoresis (0.8% w/v) in TAE buffer.

**Cell culture and DNA transfection assays.** Ovarian cells of wild-type Chinese hamsters (CHO-k1; no. ATCC CCL-61) were cultured in Dulbecco's modified Eagle's medium (DMEM) supplemented with 10% (v/v) of foetal bovine serum, 2 mM glutamine plus, 100 U/ml of penicillin and 0.1 µg/ml of streptomycin. Prior to the transfection, the cells were seeded in 24 well plates and incubated for 24 h, to reach an 80%-90% cell confluence. For the transfection experiments, plasmid pEGFP-N3 (1 µg) was mixed with the corresponding CD derivative at N/P ratios ranging between 0.1 and 20, at room temperature for 20 minutes, in a final volume of 20 µl. The mixture was diluted to 0.5 ml with DMEM without serum and added to each well. The untransfected cells and the naked pEGFP-N3 were used as negative controls. As a positive control, a transfection experiment was performed using 1 µl of Lipofectamine^{™} 2000 (Invitrogen) and 1.0 µg of DNA, following the manufacturer's instructions. The cells were incubated with the DNA-polycationic amphiphilic βCD mixtures for 18 h; subsequently, the transfection medium was eliminated and the cells were further cultured in DMEM medium plus 10% FBS for an additional 48-h period.

**Fluorescence and protein assays.** The transfected cells were washed three times with PBS and 600 µl of 0.5% triton X-100 in PBS. The plates were stirred for 10 min at room temperature, the lysate was recovered and the fluorescence was quantified in a Shimadzu RF-5301 PC fluorimeter with an excitation wavelength of 460 nm (5 nm) and an emission wavelength of 510 nm (10 nm). The protein concentration was measured using the Bio-Rad protein assay (Hercules, CA, USA).

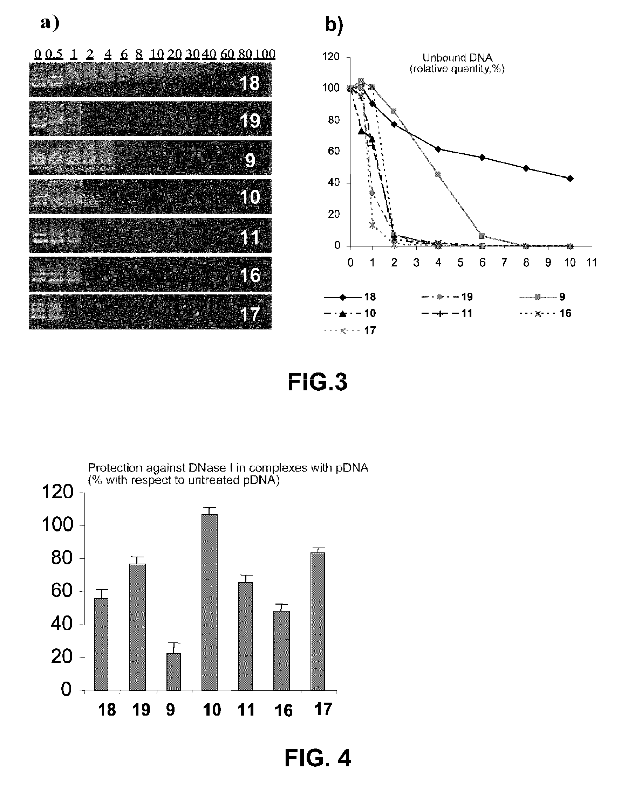

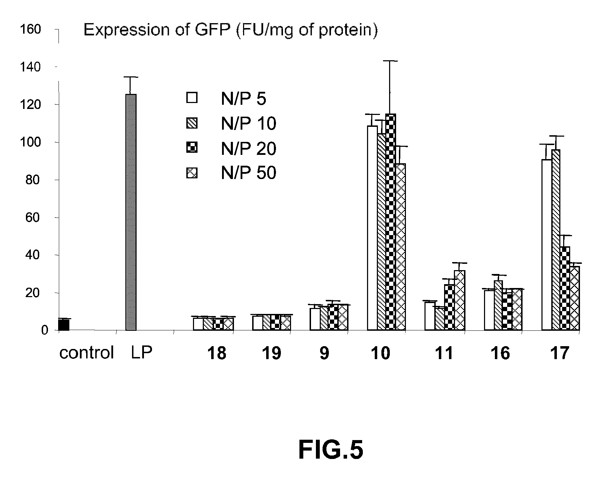

The data on the relative complexation capacities of the pDNA (pEGFP-N3 plasmid that encodes GFP) of the new polycationic amphiphilic CDs at different N/P values (ratio of protonatable nitrogens in the CD vehicle/phosphate groups in the plasmid), the degree of protection of the plasmid in the corresponding complexes against the action of DNase and the efficiency in promoting transfection (CHO-k1 cells) are shown in Figures 3, 4 and 5, respectively. The βCD derivatives modified on a single face, **18** and **19,** were used as control compounds to evaluate the influence of amphiphilicity on the interaction between the polycationic amphiphilic macrocycles of the invention and pDNA. Although dendritic architecture **19** is much more efficient than heptamine derivative **18** in compaction of the pDNA and protection against degradation by DNase, neither of them was able to promote gene transfection to a significant degree.

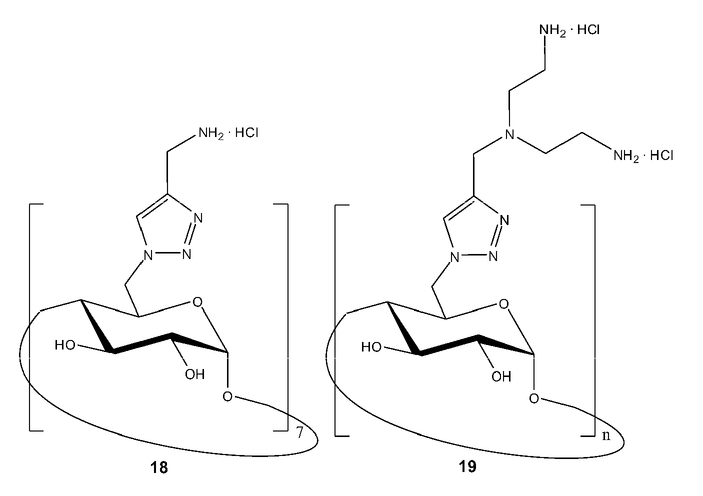

Heptacationic tetradecahexanoylated derivative **9** presented improved complexation capacities of pDNA as compared to non-amphiphilic analogue 18. Nonetheless, the plasmid still remains accessible to DNase in the corresponding **9**:pDNA complexes and the transfection levels remained very low. The benefits conferred by amphiphilicity are much more evident when the behaviour was compared within the series of branched polycationic amphiphilic derivatives **19, 10** and **11.** Compound **10,** which has hexanoate chains, led to stable **10**:pDNA complexes that guaranteed complete protection of pDNA against the environment for N/P values greater than **4**. Moreover, they showed surprisingly high transfection efficiencies, analogous to those obtained using Lipofectamine^{™} 2000. The increase in the length of the acyl chains from hexanoate to tetradecanoate did not modify the complexation efficiency, as may be observed in the data for **11,** but it drastically reduced the transfection capacity, which stresses the importance of being able to adjust the hydrophobic-hydrophilic balance, not only in order to optimise the formation of nanoparticles of polycationic amphiphilic CD-DNA, but also for the additional processes that lead to the internalisation of cells and gene expression. The ease and efficacy wherewith this adjustment may be made in the case of compounds with formula (I) is a significant characteristic of the present invention.

Compounds **16** and **17** reproduce the decoration of polyaminotriazole/tetradeca-*O*-hexanoyl of **10** and **11** in the primary face secondary faces of βCD, respectively, but incorporate a spacer arm between the triazole rings and the macrocyclic core that endows the system with much greater flexibility. An analysis of the corresponding data indicated moderate increases in the stability of the **16**:pDNA complex, in the efficacy of the protection of pDNA against the action of DNase and in the transfection efficiency of DNA as compared to the corresponding **10**:pDNA complex. Unexpectedly, the data for **11** and **17** did not show the same trend; in this case, the rigid motif was more advantageous than the flexible architecture.

Overall, the examples shown illustrate the usefulness of the polycationic amphiphilic macrocycles of the invention as transport systems for molecules and biomolecules to the interior of cells, especially polyanionic compounds such as nucleic acids. A very important aspect of the invention is that the polycationic amphiphilic macrocycles thereof may be prepared by combining very effective synthetic processes, which make it possible to access fully homogeneous macromolecules. The bi-directional strategy aimed at molecular diversity, illustrated in the above examples, is especially adequate to perform structure-activity and optimisation studies. It has been shown that molecular flexibility, the charge density and the hydrophobic-hydrophilic balance are critical parameters that may be accurately adjusted in the compounds of the invention.

## Claims

1. Compound with formula (I) 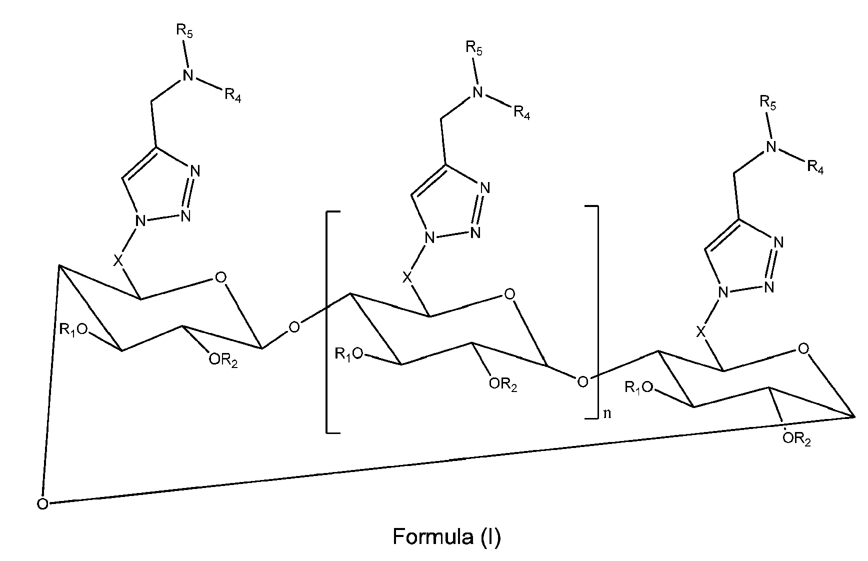 where:
n is 4, 5 or 6;
R₁ and R₂ are independently selected from hydrogen, C₁-C₂₀ alkyl or C₂-C₂₀ acyl; where at least one of R¹ and R² is other than H;
X is selected from -CH₂- or -CH₂YR₃;
where Y is selected from oxygen or sulfur, R³ is -(CH₂)ₚ-NH-CO-(CH₂)_{q}-, where p and q may independently have a value ranging between 1 and 10.
R₄ and R₅ are independently selected from hydrogen, -(CH₂)ᵣ-[(NH)-(CH₂)ₛ]ₜ-NH-R₆; where r may have a value ranging between 1 and 10, s may have a value ranging between 2 and 10, t may have a value ranging between 0 and 4;
R₆ is selected from H, a fluorochrome or a G group that comprises a ligand capable of being recognised by a cellular receptor,
or the organic or inorganic salts thereof.

2. Compound with formula (I) according to claim 1, where R¹ and R² are C₂-C₁₄ alkyl or acyl.

3. Compound with formula (I) according to any of claims 1 or 2, where X is - CH2-.

4. Compound with formula (I) according to any of claims 1 to 3, where X es -CH₂YR³, Y is sulfur, R³ is -(CH₂)ₚ-NH-CO-(CH₂)_{q}-, p is 2 and q is 1.

5. Compound with formula (I) according to any of claims 1 to 4, where R⁴ and R⁵ are -(CH₂)ᵣ-[(NH)-(CH₂)ₛ]ₜ-NH₂, where r has a value of 1, s has a value of 2 and t has a value ranging between 0 and 4.

6. Compound with formula (I) according to any of claims 1 to 5, where the salt thereof is selected from the group formed by acetate, trifluoroacetate, propionate, benzoate, methanesulfonate and trifluoromethanesulfonate.

7. Compound with formula (I) according to any of claims 1 to 5, where the salt thereof is selected from the group formed by chloride, bromide and iodide.

8. Compound with formula (I) according to claim 6, where the salt thereof is chloride.

9. Compound with formula (I) according to any of claims 1 to 8, where n is 5.

10. Compound with formula (I) according to claim 1, which is selected from the following group:
- Heptakis[6-(4-aminomethyl-1*H*-1,2,3-triazole-1-yl)-6-deoxy-2,3-di-*O-*hexanoyl]cyclomaltoheptaose
- Heptakis[6-(4-(2,2-diaminoethylaminomethyl)-1*H*-1,2,3-triazole-1-yl)-6-deoxy-2,3-di-*O*-hexanoyl]cyclomaltoheptaose
- Heptakis[6-(4-(2,2-diaminoethylaminomethyl)-1*H*-1,2,3-triazole-1-yl)-6-deoxy-2,3-di-*O*-myristoyl]cyclomaltoheptaose
- Heptakis[6-(2-(4-aminomethyl-1*H*-1,2,3-triazole-1-yl)acetamidoethylthio)-6-deoxy-2,3-di-*O*-hexanoyl]cyclomaltoheptaose
- Heptakis[6-(2-(4-(2,2-diaminoethylaminomethyl)-1*H*-1,2,3-triazole-1-yl) acetamidoethylthio)-6-deoxy-2,3-di-*O*-hexanoyl]cyclomaltoheptaose

11. Compound with formula (I) according to any of claims 1 to 10, where R₆ is a fluorochrome.

12. Compound with formula (I) according to any of claims 1 to 10, where R₆ is a G group that comprises a ligand that is selected from a mono- or oligosaccharide, a monoclonal antibody or a folic acid derivative.

13. Compound according to the preceding claim, where the number of G groups in the final compound ranges between 1 and 7.

14. Compound with formula (I) according to any of claims 12 or 13, where the mono- or oligosaccharide is selected from mannose, galactose, glucose, N-acetylglucosamine, sialic acid, lactose or an oligosaccharide formed from any of them or the combinations thereof.

15. Compound according to any of claims 12 to 14, where R₆ is a G group with the following formula:

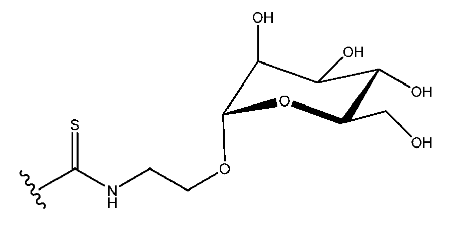

16. Use of the compound with formula (I) according to any of claims 1 to 15, as transporters of, at least, one molecule to the interior of a prokaryotic or eukaryotic cell.

17. Use of the compound according to claim 16, where the molecule belongs to the list that comprises: nucleic acid, peptide nucleic acid, peptide, protein, glycoprotein, carbohydrate, lipid or glycolipid.

18. Use of the compound according to claim 17, where the molecule is a nucleic acid.

19. Use of the compound according to claim 18, where the molecule is DNA.

20. Use of the compound according to any of claims 16 to 19, where the cell is eukaryotic.

21. Use of the compound with formula (I) according to any of claims 1 to 15, for the preparation of a medicament.

22. Use of the compound with formula (I) according to any of claims 1 to 15, for gene therapy.

23. Pharmaceutical composition that comprises the compound with formula (I) according to any of claims 1 to 15.

24. Pharmaceutical composition according to claim 23, which further comprises a pharmaceutically acceptable vehicle.

25. Pharmaceutical composition according to any of claims 23 or 24, which further comprises another active principle.
